# EUROPÄISCHE PATENTSCHRIFT

(11) **EP 4 025 839 B1**
(45) Veröffentlichungstag und Bekanntmachung des Hinweises auf die Patenterteilung: **25.10.2023**
(21) Anmeldenummer: 20772221.6
(22) Anmeldetag: 04.09.2020
(51) Int. Cl.: F24F 8/20, F24F 8/30, F24F 8/40, F24F 110/70, F24F 110/20, F24F 110/50, F24F 110/64, F24F 110/66

(54) **EINRICHTUNG ZUR LUFTBEHANDLUNG MIT IONISATIONSMODUL**
DEVICE FOR THE TREATMENT OF AIR WITH AN IONIZING MODULE
DISPOSITIF POUR LE TRAITEMENT D'AIR À L'AIDE D'UN MODULE D'IONISATION

(30) Priorität: 05.09.2019 DE 102019123885
(43) Veröffentlichungstag der Anmeldung: 13.07.2022
(73) Patentinhaber: Ionair AG, 6014 Luzern (CH)
(72) Erfinder: WEIBEL, Beda, 6014 Luzern (CH)
(74) Vertreter: Kailuweit & Uhlemann Patentanwälte Partnerschaft mbB
(86) Internationale Anmeldenummer: PCT/EP2020/074730
(87) Internationale Veröffentlichungsnummer: WO 2021/043963

(56) Entgegenhaltungen:
- CH-A1- 713 394
- DE-A1- 4 334 956
- DE-A1- 10 007 523
- DE-U1-202004 021 032

## Beschreibung

Die Erfindung betrifft Einrichtungen zur Luftbehandlung wenigstens eines Raumes.

Die Luft in einem Raum ist mit Einrichtungen zur Ionisierung behandelbar. Dabei werden Bakterien und andere Keime abgetötet und Großmoleküle in kleinmolekulare Fragmente aufgespalten. Komplexe und große Moleküle sind unter anderem Geruchsstoffe, so dass mit einer Luftionisation eine Geruchsbelastung unterdrückt werden kann. Weiterhin können auch Mikroorganismen in der Luft wirksam reduziert werden.

Bei Einrichtungen zur Ionisation werden elektrische Felder zwischen zwei Elektroden mit Spannungspotenzialen ausgenutzt, um durch Gasentladungen Ionen durch Stoßionisationen zu erzeugen. Dazu können bekannte lonisierungsröhren in Form von Glasröhren mit einer Innenelektrode und einer Außenelektrode eingesetzt werden. Wird eine für die Gasentladung ausreichend hohe elektrische Spannung angelegt, bildet das Glas der Wandung ein Dielektrikum, in dem ein großes elektrisches Feld vorhanden ist. Die durchströmende Luft wird mit Ionen angereichert.

Durch die Druckschrift DE 43 34 956 C2 sind ein Verfahren zur Luftbehandlung mit Ionen und eine Vorrichtung zur Durchführung des Verfahrens bekannt, wobei die Langzeitstabilität eines Ionisationsapparates erhöht wird. Hauptaugenmerk gilt der Vermeidung einer erhöhten Ozonerzeugung. Dazu werden Sensoren in Form eines Luftqualitätssensors, eines Luftströmungsfühlers und eines Luftfeuchtefühlers eingesetzt. Beim Auftreten sowohl äußerer Störquellen wie zum Beispiel bei Smog, einer Inversionswetterlage, Gewitter, äußere Energiefelder als auch innerer Störquellen durch den Betrieb elektrischer Geräte kann die Belastung an Ozon in der Zuluft in unerwünschtem Maß ansteigen und zu einer Grenzwertüberschreitung führen.

In der Druckschrift DE 100 07 523A1 wird ein Verfahren zur Luftbehandlung mit Ionen sowie eine Vorrichtung zur Durchführung des Verfahrens beschrieben, wobei zusätzlich ein Ozonsensor zur Bestimmung des Ozongehaltes eingesetzt wird.

CH 713 394 A1 offenbart einen Elektrofilter, wobei ein Zusammenhang zwischen der Verweilzeit der ionisierten Partikel in der Kollektorstufe des Elektrofilters und der elektrischen Feldstärke genannt wird.

DE 20 2004 021032 U1 offenbart eine Anordnung zur Beeinflussung und Behandlung der Luft wenigstens eines Raumes durch sowohl Temperieren als auch Ionisation der Zuluft. Sie weist unter anderem auf: Zuluftleitung, lonisationsmodul mit mehreren Ionisationsröhren, Luftströmungsfühler sowie Luftfeuchtefühler. Nachteil ist, dass eine sich verringernde lonisationsleistung lange Zeit unerkannt bleibt.

Der im Patentanspruch 1 angegebenen Erfindung liegt die Aufgabe zugrunde, eine bakterien-, keim- und geruchsarme Luft in wenigstens einem Raum bereitzustellen.

Diese Aufgabe wird mit den im Patentanspruch 1 aufgeführten Merkmalen gelöst.

Die Einrichtungen zur Luftbehandlung wenigstens eines Raumes zeichnen sich insbesondere dadurch aus, dass eine bakterien-, keim- und geruchsarme Luft in wenigstens einen Raum bereitstellbar ist.

Im Sinne der Erfindung weist die Einrichtung zur Luftbehandlung mindestens eine behandelte Luft zuführende Zuluftleitung mit jeweils wenigstens einer Einrichtung zur Luftaufbereitung mit wenigstens einem lonisationsmodul mit mehreren Ionisationsröhren, einem Luftströmungssensor und einem Luftfeuchtesensor auf. Erfindungsgemäß besitzt mindestens eine Abluftleitung wenigstens einen Luftgütesensor. Im Sinne der Erfindung sind weiterhin mindestens eine in Strömungsrichtung der Zuluft vor dem lonisationsmodul angeordnete Staubmesseinrichtung und/oder wenigstens eine in der Abluftleitung angeordnete Staubmesseinrichtung vorhanden. Erfindungsgemäß ist ein Datenverarbeitungssystem mit dem lonisationsmodul, dem Luftströmungssensor, dem Luftfeuchtesensor, dem Luftgütesensor und der Staubmesseinrichtung vor dem lonisationsmodul und/oder der Staubmesseinrichtung der Abluftleitung zur Erhöhung oder Verringerung der lonisationsintensität entsprechend den Messwerten des Luftströmungssensors, des Luftfeuchtesensors, des Luftgütesensors und der Staubmesseinrichtung vor dem lonisationsmodul und/oder der Staubmesseinrichtung der Abluftleitung verbunden. Im Sinne der Erfindung ist oder sind darüber hinaus wenigstens eine den Laststrom des lonisationsmoduls und/oder mindestens einer Ionisationsröhre des lonisationsmoduls messende Einrichtung mit dem Datenverarbeitungssystem verbunden, wobei das Datenverarbeitungssystem bei einer Laststromänderung des lonisationsmoduls oder wenigstens einer Ionisationsröhre und/oder bei einer Laststromänderung des lonisationsmoduls oder wenigstens einer Ionisationsröhre in Abhängigkeit der Messwerte der Staubmesseinrichtung einen Betriebszustand des lonisationsmoduls zuordnet.

Mit Hilfe der Einrichtung wird eine verbesserte Raumluft für den Raumnutzer erzielt. Das erfolgt unter Berücksichtigung der Parameter, die die lonisationswirkung beeinflussen und unter Berücksichtigung geltender Grenzwerte. Dazu kann Außenluft und/oder Umluft als Abluft des Raumes über die Einrichtung zur Luftaufbereitung nach der Luftaufbereitung als Zuluft über die Zuluftleitung in den Raum gelangen. Der Raumnutzer genießt dadurch reduzierte VOC-Konzentrationen, reduzierte Bakterien und Keimzahl, sowie reduzierte PM-Konzentrationen (PM - Particulate Matter, gleich Feinstaub) in der geatmeten Raumluft. VOC sind dabei flüchtige organische Verbindungen (volatile organic compounds - VOC), beispielsweise Tabakrauch, Küchendunst, Ausdünstungen von Menschen oder Ausgasungen von Materialien wie zum Beispiel aus Möbeln, Teppichen, Klebern und dergleichen. Mit der Einrichtung ist damit die Raumlufthygiene gesteigert.

Der verwendete Luftgütesensor kann dazu ein Metalloxid-Halbleitergassensor sein, der auch zur Erfassung von flüchtigen organischen Substanzen/Verbindungen (VOC) einsetzbar ist.

Mit der wenigstens einen den Laststrom des lonisationsmoduls und/oder mindestens einer Ionisationsröhre des lonisationsmoduls messenden Einrichtung ist oder sind vorteilhafterweise eine durch Alterung sich verringernde lonisationsleistung wenigstens einer Ionisationsröhre als Betriebszustand des lonisationsmoduls feststellbar. Der Laststrom ist dazu der durch das lonisationsmodul und/oder die mindestens eine Ionisationsröhre während des Betriebs fließende elektrische Strom. In Verbindung mit der Staubmesseinrichtung ist eine sich verringernde lonisationsleistung bei Verschmutzung wenigstens einer Ionisationsröhre als Betriebszustand des lonisationsmoduls ermittelbar. Dazu wird eine Laststromänderung gegenüber einem nominellen Laststrom ermittelt. Der nominelle Laststrom kann der Laststrom bei Erstinbetriebnahme oder Wiederinbetriebnahme (z.B. nach Wartung) der Einrichtung zur Luftbehandlung sein.

In Ausführungsformen ist die Einrichtung zur Luftbehandlung als Baueinheit einer raumlufttechnischen Anlage ausgebildet. Darüber hinaus kann die Einrichtung zur Luftbehandlung auch eine Baueinheit eines Raumluftgeräts sein.

Der Betriebszustand kann dazu als wenigstens ein Kennwert in einer Grafik und/oder einer Tabelle und/oder einer Farbskala zugeordnet und dargestellt werden. Vorteilhafterweise wird dazu die jeweilige Zeit zugeordnet.

Vorteilhafte Ausgestaltungen der Erfindung sind in den Patentansprüchen 2 bis 11 angegeben.

Gemäß einer Ausführungsform des Erfindungsgegenstands ist die den Laststrom einer Ionisationsröhre des lonisationsmoduls messende Einrichtung mit dem Datenverarbeitungssystem verbunden, welches ein bei einer Laststromänderung der Ionisationsröhre und/oder ein bei einer Laststromänderung der Ionisationsröhre in Abhängigkeit der Messwerte der Staubmesseinrichtung vor dem lonisationsmodul und/oder der Staubmesseinrichtung der Abluftleitung diese Änderung dem Betriebszustand der Ionisationsröhre zuordnendes Datenverarbeitungssystem ist.

Die Abluftleitung des Raumes ist gemäß einer weiteren Ausführungsform des Erfindungsgegenstands mit einer verbrauchte Luft abführenden Leitung und/oder mit einer Außenluft zuführenden Leitung verbunden. Weiterhin ist die Außenluft zuführende Leitung mit der Einrichtung zur Luftaufbereitung verbunden.

Gemäß einer bevorzugten Ausführungsform weisen die verbrauchte Luft abführende Leitung eine erste Armatur und die Außenluft zuführende Leitung eine zweite Armatur auf. Die Betätigungseinrichtungen der Armaturen sind zum Verändern des jeweiligen Luftstromes mit dem Datenverarbeitungssystem verbunden. Damit kann unter anderem Außenluft zugeführt werden oder ein Umluftbetrieb eingestellt werden. In Ausführungsformen sind die Armaturen als Ventile oder Klappen ausgebildet.

Die Außenluft zuführende Leitung besitzt nach einer weiteren bevorzugten Ausführungsform eine weitere Staubmesseinrichtung zur Messung des Staubs der Außenluft. Die Staubmesseinrichtung in der Außenluft zuführenden Leitung ist mit dem Datenverarbeitungssystem verbunden, welches bei Auftreten oder erhöhtem Auftreten von Staub in der Außenluft die lonisationsleistung des lonisationsmoduls und/oder die Luftströmung in Abhängigkeit des Wertes der Staubmesseinrichtung in der Außenluft zuführenden Leitung vergrößert.

Der Luftströmungssensor, der Luftfeuchtesensor, der Luftgütesensor und die Staubmesseinrichtung vor dem lonisationsmodul und/oder die Staubmesseinrichtung der Abluftleitung sind gemäß einer weiteren Ausführungsform des Erfindungsgegenstands mit dem Datenverarbeitungssystem verbunden, welches in Abhängigkeit der Raumgröße und von Messwerten des Luftströmungssensors, des Luftfeuchtesensors, des Luftgütesensors und der Staubmesseinrichtung vor dem lonisationsmodul und/oder der Staubmesseinrichtung der Abluftleitung eine Raumluftkonzentration als ein Modell mit Gewichten der Luftströmung, der Luftfeuchte, der Luftgüte und des Staubs ermittelt.

Wenigstens ein Ozonsensor ist oder sind gemäß einer weiteren Ausführungsform des Erfindungsgegenstands in der Zuluftleitung und/oder in der Abluftleitung und/oder im Raum und/oder in der Außenluft zuführenden Leitung angeordnet. Der Ozonsensor oder die Ozonsensoren ist oder sind mit dem Datenverarbeitungssystem verbunden, welches aus den Messwerten des Luftfeuchtesensors, des Luftströmungssensors, des Luftgütesensors und der Staubmesseinrichtung vor dem lonisationsmodul und/oder der Staubmesseinrichtung der Abluftleitung und dem Ozonsensor oder den Ozonsensoren entsprechend den Gewichten der Luftfeuchte, der Luftströmung, der Luftgüte und des Staubs und eines Ozongrenzwertes die lonisationsintensität bestimmt und bei einer Veränderung der lonisationsintensität die Ionisationsleistung so ändert, dass eine Kompensation und entsprechend der Veränderung eine darüber hinausgehende Verringerung oder Erhöhung der lonisationsintensität erfolgt ohne dass ein beeinträchtigender oder schädigender Ozongrenzwert überschritten wird.

Gemäß einer weiteren Ausführungsform des Erfindungsgegenstands ist oder sind wenigstens ein Kohlendioxidsensor in der Zuluftleitung und/oder in der Abluftleitung und/oder im Raum angeordnet, der oder die weiterhin mit dem Datenverarbeitungssystem verbunden ist oder sind. Das Datenverarbeitungssystem ist ein bei einer Veränderung des Kohlendioxids die Zuluft und/oder die Luftströmung unter Berücksichtigung der lonisationsintensität so änderndes Datenverarbeitungssystem, dass in Abhängigkeit der Messwerte des Luftfeuchtesensors, des Luftströmungssensors, des Luftgütesensors und der Staubmesseinrichtung vor dem lonisationsmodul und/oder der Staubmesseinrichtung der Abluftleitung die lonisationsintensität bestimmt und bei einer Veränderung eine Verringerung oder Erhöhung der lonisationsintensität erfolgt und ein Kohlendioxidgrenzwert eingehalten wird. Das erfolgt mit Zuführung von Außenluft, wozu Armaturen vorgesehen sein können.

Das Ionisationsröhren aufweisende lonisationsmodul ist gemäß einer weiteren Ausführungsform des Erfindungsgegenstands mit dem Datenverarbeitungssystem verbunden, welches die Ionisationsleistung der Ionisationsröhren mit einer Pulsweitenmodulierung (PWM) mittels Schalten von Perioden kleiner oder größer 100 Perioden vom Stromnetz steuert. In bevorzugten Ausführungsformen können dies Perioden größer 0 und kleiner/gleich 100 Perioden sein. In weiteren Ausführungsformen können die Perioden größer 0 und kleiner/gleich 200 Perioden sein. Bei einer Laststromänderung wenigstens einer Ionisationsröhre wird die Pulsweitenmodulierung entsprechend der Laststromänderung geändert.

Die jeweils den Laststrom einer Ionisationsröhre des lonisationsmoduls messende Einrichtung und/oder die den Laststrom der Ionisationsröhren des lonisationsmoduls messende Einrichtung ist oder sind gemäß einer weiteren Ausführungsform des Erfindungsgegenstands mit dem Datenverarbeitungssystem so verbunden, dass bei einer Laststromänderung wenigstens einer Ionisationsröhre die Lastströme der anderen Ionisationsröhren gegenüber einem bekannten nominellen Laststrom geändert werden.

Das Datenverarbeitungssystem ist gemäß einer weiteren Ausführungsform des Erfindungsgegenstands mit einem Sender elektromagnetischer Wellen und/oder Sendeempfänger und/oder mit einem Datennetz und/oder einem Rechnernetz verbunden. Die Messwerte des Luftströmungssensors, des Luftfeuchtesensors, des Luftgütesensors und der Staubmesseinrichtung sind so fernabfragbar.

Für die Realisierung der Erfindung ist es auch zweckmäßig, die vorbeschriebenen erfindungsgemäßen Ausgestaltungen, Ausführungsformen und Merkmale der Ansprüche in jeder Anordnung miteinander zu kombinieren.

Ein Ausführungsbeispiel der Erfindung ist in den Zeichnungen jeweils prinzipiell dargestellt und wird im Folgenden näher beschrieben.

Es zeigen:
- Fig. 1: eine Einrichtung zur Luftbehandlung wenigstens eines Raumes und
- Fig. 2: eine Einrichtung zur Luftbehandlung wenigstens eines Raumes mit zusätzlichen Ozonsensoren, einem zusätzlichen Kohlendioxidsensor und einer weiteren Staubmesseinrichtung.

Eine Einrichtung zur Luftbehandlung wenigstens eines Raumes 1 besteht im Wesentlichen aus einer behandelte Luft zuführenden Zuluftleitung 2 mit einer Einrichtung zur Luftaufbereitung 3 mit einem lonisationsmodul 4 mit mehreren Ionisationsröhren, einem Luftströmungssensor 5 und einem Luftfeuchtesensor 6, einer Abluftleitung 7 mit einem Luftgütesensor 8, einer vor dem lonisationsmodul 4 angeordneten Staubmesseinrichtung 9 und wenigstens einer in der Abluftleitung 7 angeordneten Staubmesseinrichtung 11, einem mit dem lonisationsmodul 4, dem Luftströmungssensor 5, dem Luftfeuchtesensor 6, dem Luftgütesensor 8 und der Staubmesseinrichtung 9 vor dem lonisationsmodul 4 und der Staubmesseinrichtung 11 der Abluftleitung 7 verbundenen Datenverarbeitungssystem 10.

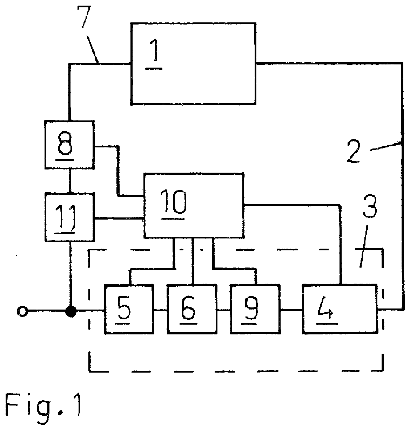

Die Fig. 1 zeigt eine Einrichtung zur Luftbehandlung wenigstens eines Raumes 1 in einer prinzipiellen Darstellung.

Die Staubmesseinrichtung 9 ist so ausgebildet, dass insbesondere auch Feinstaub erfassbar ist. Einrichtungen zur Staubmessung und Feinstaubmessung sind bekannt.

Außenluft und/oder Umluft als Abluft des Raumes 1 gelangt über die Einrichtung zur Luftaufbereitung 3 als Zuluft über die Zuluftleitung 2 in den Raum 1. Die Einrichtung zur Luftaufbereitung 3 besitzt das lonisationsmodul 4 mit mehreren Ionisationsröhren, den Luftströmungssensor 5, den Luftfeuchtesensor 6 und die Staubmesseinrichtung 9 vor dem lonisationsmodul 4. Die Abluftleitung 7 zur Abfuhr von Raumluft weist den Luftgütesensor 8 und die Staubmesseinrichtung 11 der Abluftleitung 7 auf. Der Luftgütesensor 8 kann insbesondere ein Metalloxid-Halbleitergassensor auch für flüchtige organische Substanzen/Verbindungen VOC sein. Die Abluftleitung 7 stellt so gleichzeitig eine Umluftleitung dar, wobei Abluft als Zuluft wieder zugeführt wird. Dazu ist diese mit der Einrichtung 3 zur Luftbehandlung verbunden. Das lonisationsmodul 4, der Luftströmungssensor 5, der Luftfeuchtesensor 6, der Luftgütesensor 8 und die Staubmesseinrichtungen 9, 11 sind mit dem Datenverarbeitungssystem 10 verbunden. Eine so ausgebildete Einrichtung zur Luftbehandlung ist eine Baueinheit einer raumlufttechnischen Anlage.

Das Datenverarbeitungssystem 10 ist so ausgebildet, dass die lonisationsintensität entsprechend der Messwerte des Luftströmungssensors 5, des Luftfeuchtesensors 6, des Luftgütesensors 8 und der Staubmesseinrichtungen 9, 11 verringert oder vergrößert wird, so dass sich bei Erhöhung der Luftfeuchte und/oder des Staubs sowie bei Verringerung der Luftgüte die lonisationsintensität und/oder die Luftströmung vergrößert.

Der Luftströmungssensor 5, der Luftfeuchtesensor 6, der Luftgütesensor 8 und die Staubmesseinrichtungen 9, 11 sind dazu mit dem Datenverarbeitungssystem 10 verbunden, welches in Abhängigkeit der Raumgröße und von Messwerten des Luftströmungssensors 5, des Luftfeuchtesensors 6, des Luftgütesensors 8 und der Staubmesseinrichtung 9, 11 eine Raumluftkonzentration als ein Modell mit Gewichten der Luftströmung, der Luftfeuchte, der Luftgüte und des Staubs ermittelt. Weiterhin ist das Datenverarbeitungssystem 10 ein aus dem Modell der Raumluftkonzentration die mittlere Raumluftkonzentration ermittelndes und einem Grenzwert der lonisationsintensität zuordnendes Datenverarbeitungssystem 10.

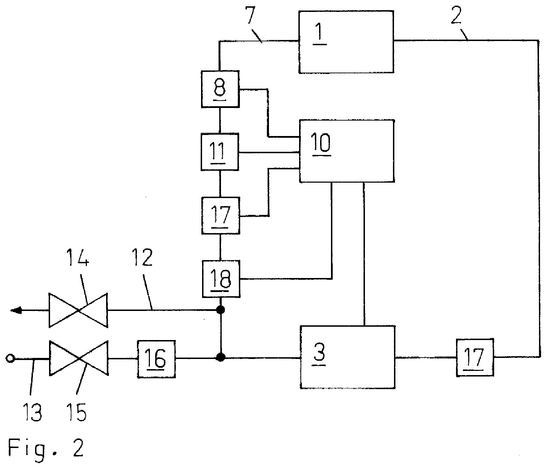

Die Fig. 2 zeigt eine Einrichtung zur Luftbehandlung wenigstens eines Raumes 1 mit gegenüber der in Fig.1 dargestellten Einrichtung zusätzlichen Ozonsensoren 17, einem Kohlendioxidsensor 18 und einer weiteren Staubmesseinrichtung 16 in einer prinzipiellen Darstellung. Auf die Bezugszeichen der in Fig. 1 dargestellten Einrichtung wird nachfolgend Bezug genommen

In einer Ausführungsform kann die Einrichtung zur Luftbehandlung wenigstens eines Raumes 1 weiterhin Ozonsensoren 17, einen Kohlendioxidsensor 18 und eine weitere Staubmesseinrichtung 16 aufweisen. Jeweils ein Ozonsensor 17 befindet sich in der Zuluftleitung 2 und der Abluftleitung 7. Darüber hinaus kann ein Ozonsensor 17 auch in einer Außenluft zuführenden Leitung 13 angeordnet sein. Der Kohlendioxidsensor 18 ist in der Abluftleitung 7 als Umluftleitung angeordnet. Die weitere Staubmesseinrichtung 16 befindet sich in der Außenluft zuführenden Leitung 13. Damit ist die Abluftleitung 7 des Raumes 1 mit der Einrichtung zur Luftaufbereitung 3 auch als Umluftleitung verbindbar. Die Abluftleitung 7 ist weiterhin mit einer verbrauchte Luft abführenden Leitung 12 in Form einer Fortluftleitung mit einer ersten Armatur 14 und der Außenluft zuführenden Leitung 13 mit einer zweiten Armatur 15 verbunden. Die Betätigungseinrichtungen der Armaturen 14, 15 sind zum Verändern des jeweiligen Luftstromes mit dem Datenverarbeitungssystem 10 verbunden. Dazu können die Armaturen 14, 15 beispielsweise Klappen sein.

Die Ozonsensoren 17 sind mit dem Datenverarbeitungssystem 10 verbunden, welches aus den Messwerten des Luftströmungssensors 5, des Luftfeuchtesensors 6, des Luftgütesensors 8, der Staubmesseinrichtungen 9, 11, 16 und den Ozonsensoren 17 entsprechend den Gewichten der Luftströmung, der Luftfeuchte, der Luftgüte, des Staubs und des Ozons die lonisationsintensität bestimmt. Bei einer Veränderung der lonisationsintensität wird mittels des Datenverarbeitungssystems 10 die Ionisationsleistung so geändert, dass eine Kompensation und entsprechend der Veränderung eine darüber hinausgehende Verringerung oder Erhöhung der lonisationsintensität erfolgt ohne dass ein beeinträchtigender oder schädigender Ozongrenzwert überschritten wird.

Insbesondere können Ozonsensoren 17 in der Zuluftleitung 2, in der Abluftleitung 7 zur Messung des Ozons in der Raumluft und in der Außenluft zuführenden Leitung 13 zur Messung des Ozons der Außenluft angeordnet sein. Falls das Ozon in der Außenluft zu hoch ist, kann so die Einrichtung zur Luftbehandlung wenigstens eines Raumes im Umluftbetrieb betrieben werden.

Der Kohlendioxidsensor 18 ist mit dem Datenverarbeitungssystem 10 so verbunden, dass bei einer Veränderung des Kohlendioxids die Zuluft und/oder die Luftströmung unter Berücksichtigung der lonisationsintensität so geändert wird, dass in Abhängigkeit der Messwerte des Luftströmungssensors 5, des Luftfeuchtesensors 6, des Luftgütesensors 8 und der Staubmesseinrichtungen 9, 11, 16 die lonisationsintensität bestimmt und bei einer Veränderung eine Verringerung oder Erhöhung der lonisationsintensität erfolgt. Dabei wird ein Kohlendioxidgrenzwert eingehalten.

Weitere mit dem Datenverarbeitungssystem 10 verbundene Kohlendioxidsensoren 18 können sich in der Zuluftleitung 2 und/oder im Raum 1 befinden.

Das lonisationsmodul 4 des Ausführungsbeispiels besitzt mehrere Ionisationsröhren. Wenigstens eine jeweils den Laststrom einer Ionisationsröhre messende Einrichtung und/oder eine den Laststrom der Ionisationsröhren messende Einrichtung ist oder sind mit dem Datenverarbeitungssystem 10 verbunden, wobei das Datenverarbeitungssystem 10 ein bei einer Laststromänderung des lonisationsmoduls oder wenigstens einer Ionisationsröhre und/oder ein bei einer Laststromänderung des lonisationsmoduls oder wenigstens einer Ionisationsröhre in Abhängigkeit der Messwerte der Staubmesseinrichtung 9 vor dem lonisationsmoduls 4 einen Betriebszustand des lonisationsmoduls 4 zuordnet.

Das Ionisationsröhren aufweisende lonisationsmodul 4 ist weiterhin mit dem Datenverarbeitungssystem 10 verbunden, welches die Ionisationsleistung der Ionisationsröhren mit einer Pulsweitenmodulierung (PWM) mittels Schalten von Perioden größer 0 und kleiner/gleich 100 Perioden vom Stromnetz steuert. Weiterhin kann wenigstens eine jeweils den Laststrom einer Ionisationsröhre des lonisationsmoduls 4 messende Einrichtung und/oder eine den Laststrom der Ionisationsröhren messende Einrichtung mit dem Datenverarbeitungssystem 10 verbunden sein, welches bei einer Laststromänderung wenigstens einer Ionisationsröhre die Lastströme der anderen Ionisationsröhren gegenüber einem bekannten nominellen Laststrom ändert. Bei einer Laststromänderung wenigstens einer Ionisationsröhre wird die Pulsweitenmodulierung entsprechend der Laststromänderung geändert.

### Bezugszeichenliste

1 Raum
2 Zuluftleitung
3 Einrichtung zur Luftaufbereitung
4 lonisationsmodul
5 Luftströmungssensor
6 Luftfeuchtesensor
7 Abluftleitung
8 Luftgütesensor
9 Staubmesseinrichtung vor dem lonisationsmodul 4
10 Datenverarbeitungssystem
11 Staubmesseinrichtung in der Abluftleitung 7
12 verbrauchte Luft abführende Leitung
13 Außenluft zuführende Leitung
14 erste Armatur
15 zweite Armatur
16 weitere Staubmesseinrichtung
17 Ozonsensor
18 Kohlendioxidsensor

## Patentansprüche

1. Einrichtung zur Luftbehandlung wenigstens eines Raumes (1) mit
- mindestens einer behandelte Luft zuführenden Zuluftleitung (2) mit jeweils wenigstens einer Einrichtung zur Luftaufbereitung (3) mit wenigstens jeweils einem lonisationsmodul (4) mit mehreren Ionisationsröhren, einem Luftströmungssensor (5) und einem Luftfeuchtesensor (6),
- mindestens einer Abluftleitung (7) mit wenigstens einem Luftgütesensor (8),
- einem mit dem lonisationsmodul (4), dem Luftströmungssensor (5), dem Luftfeuchtesensor (6), dem Luftgütesensor (8) verbundenen Datenverarbeitungssystem (10) zur Erhöhung oder Verringerung der lonisationsintensität entsprechend den Messwerten des Luftströmungssensors (5), des Luftfeuchtesensors (6), des Luftgütesensors (8) und **dadurch gekennzeichnet, dass**
- mindestens einer vor dem lonisationsmodul (4) angeordneten Staubmesseinrichtung (9) und/oder wenigstens einer in der Abluftleitung (7) angeordneten Staubmesseinrichtung (11) vorhanden sind, und
- wenigstens einer den Laststrom des lonisationsmoduls (4) und/oder mindestens einer Ionisationsröhre des lonisationsmoduls (4) messenden Einrichtung, welche mit dem Datenverarbeitungssystem (10) verbunden ist oder sind, vorhanden sind
wobei das Datenverarbeitungssystem (10) auch mit der Staubmesseinrichtung (9) vor dem lonisationsmodul (4) und/oder einer Staubmesseinrichtung (11) der Abluftleitung (7) verbunden ist, zur Erhöhung oder Verringerung der lonisationsintensität entsprechend den Messwerten der Staubmesseinrichtung (9) vor dem lonisationsmodul (4) und/oder der Staubmesseinrichtung (11) der Abluftleitung (7), wobei das Datenverarbeitungssystem (10) bei einer Laststromänderung des lonisationsmoduls (4) oder wenigstens einer Ionisationsröhre in Abhängigkeit der Messwerte der Staubmesseinrichtung (9) einen Betriebszustand des lonisationsmoduls (4) zuordnet, derart, dass eine sich verringernde lonisationsleistung bei Verschmutzung wenigstens einer Ionisationsröhre als Betriebszustand des lonisationsmoduls ermittelbar ist.

2. Einrichtung nach Patentanspruch 1, **dadurch gekennzeichnet, dass** die den Laststrom einer Ionisationsröhre des lonisationsmoduls (4) messende Einrichtung mit dem Datenverarbeitungssystem (10) verbunden ist, welches ein bei einer Laststromänderung der Ionisationsröhre und/oder ein bei einer Laststromänderung der lonisationsröhre in Abhängigkeit der Messwerte der Staubmesseinrichtung (9) vor dem lonisationsmodul (4) und/oder der Staubmesseinrichtung (11) der Abluftleitung (7) diese Änderung dem Betriebszustand der Ionisationsröhre zuordnendes Datenverarbeitungssystem (10) ist.

3. Einrichtung nach Patentanspruch 1 oder 2, **dadurch gekennzeichnet, dass** die Abluftleitung (7) des Raumes (1) mit einer verbrauchte Luft abführenden Leitung (12) und/oder mit einer Außenluft zuführenden Leitung (13) verbunden ist und dass die Außenluft zuführende Leitung (13) mit der Einrichtung zur Luftaufbereitung (3) verbunden ist.

4. Einrichtung nach Patentanspruch 3, **dadurch gekennzeichnet, dass** die verbrauchte Luft abführende Leitung (12) eine erste Armatur (14) und die Außenluft zuführende Leitung (13) eine zweite Armatur (15) aufweisen und dass die Betätigungseinrichtungen der Armaturen (14, 15) zum Verändern des jeweiligen Luftstromes mit dem Datenverarbeitungssystem (10) verbunden sind.

5. Einrichtung nach Patentanspruch 3 oder 4, **dadurch gekennzeichnet, dass** die Außenluft zuführende Leitung (13) eine weitere Staubmesseinrichtung (16) zur Messung des Staubs der Außenluft besitzt und dass die Staubmesseinrichtung (16) in der Außenluft zuführenden Leitung (13) mit dem Datenverarbeitungssystem (10) verbunden ist, welches bei Auftreten oder erhöhtem Auftreten von Staub in der Außenluft die lonisationsleistung des lonisationsmoduls und/oder die Luftströmung in Abhängigkeit des Wertes der Staubmesseinrichtung (16) in der Außenluft zuführenden Leitung (13) vergrößert.

6. Einrichtung nach einem der vorangegangenen Patentansprüche, **dadurch gekennzeichnet, dass** der Luftströmungssensor (5), der Luftfeuchtesensor (6), der Luftgütesensor (8) und die Staubmesseinrichtung (9) vor dem lonisationsmodul (4) und/oder die Staubmesseinrichtung (11) der Abluftleitung (7) mit dem Datenverarbeitungssystem (10) verbunden sind, welches in Abhängigkeit der Raumgröße und von Messwerten des Luftströmungssensors (5), des Luftfeuchtesensors (6), des Luftgütesensors (8) und der Staubmesseinrichtung (9) vor dem lonisationsmodul (4) und/oder der Staubmesseinrichtung (11) der Abluftleitung (7) eine Raumluftkonzentration als ein Modell mit Gewichten der Luftströmung, der Luftfeuchte, der Luftgüte und des Staubs ermittelt.

7. Einrichtung nach einem der vorangegangenen Patentansprüche, **dadurch gekennzeichnet, dass** wenigstens ein Ozonsensor (17) in der Zuluftleitung (2) und/oder in der Abluftleitung (7) und/oder im Raum (1) und/oder in der Außenluft zuführenden Leitung (13) angeordnet ist oder sind und dass der Ozonsensor (17) oder die Ozonsensoren (17) mit dem Datenverarbeitungssystem (10) verbunden ist oder sind, welches aus den Messwerten des Luftfeuchtesensors (6), des Luftströmungssensors (5), des Luftgütesensors (8) und der Staubmesseinrichtung (9) vor dem lonisationsmodul (4) und/oder der Staubmesseinrichtung (11) der Abluftleitung (7) und dem Ozonsensor (17) oder den Ozonsensoren (17) entsprechend den Gewichten der Luftfeuchte, der Luftströmung, der Luftgüte und des Staubs und eines Ozongrenzwertes die lonisationsintensität bestimmt und bei einer Veränderung der lonisationsintensität die Ionisationsleistung so ändert, dass eine Kompensation und entsprechend der Veränderung eine darüber hinausgehende Verringerung oder Erhöhung der lonisationsintensität erfolgt, ohne dass ein beeinträchtigender oder schädigender Ozongrenzwert überschritten wird.

8. Einrichtung nach einem der vorangegangenen Patentansprüche, **dadurch gekennzeichnet, dass** wenigstens ein Kohlendioxidsensor (18) in der Zuluftleitung (2) und/oder in der Abluftleitung (7) und/oder im Raum (1) angeordnet ist oder sind und dass der Kohlendioxidsensor (18) oder die Kohlendioxidsensoren (18) mit dem Datenverarbeitungssystem (10) verbunden ist oder sind, welches bei einer Veränderung des Kohlendioxids die Zuluft und/oder die Luftströmung unter Berücksichtigung der lonisationsintensität so ändert, dass in Abhängigkeit der Messwerte des Luftfeuchtesensors (6), des Luftströmungssensors (5), des Luftgütesensors (8) und der Staubmesseinrichtung (9) vor dem lonisationsmodul (4) und/oder der Staubmesseinrichtung (11) der Abluftleitung (7) die lonisationsintensität bestimmt und bei einer Veränderung eine Verringerung oder Erhöhung der lonisationsintensität erfolgt und ein Kohlendioxidgrenzwert eingehalten wird.

9. Einrichtung nach einem der vorangegangenen Patentansprüche, **dadurch gekennzeichnet, dass** das Ionisationsröhren aufweisende lonisationsmodul (4) mit dem Datenverarbeitungssystem (10) verbunden ist, welches die Ionisationsleistung der Ionisationsröhren mit einer Pulsweitenmodulierung (PWM) mittels Schalten von Perioden vom Stromnetz steuert.

10. Einrichtung nach einem der vorangegangenen Patentansprüche, **dadurch gekennzeichnet, dass** die jeweils den Laststrom einer Ionisationsröhre des lonisationsmoduls (4) messende Einrichtung und/oder die den Laststrom der Ionisationsröhren des lonisationsmoduls (4) messende Einrichtung mit dem Datenverarbeitungssystem (10) so verbunden ist oder sind, dass bei einer Laststromänderung wenigstens einer lonisationsröhre die Lastströme der anderen Ionisationsröhren gegenüber einem bekannten nominellen Laststrom geändert werden.

11. Einrichtung nach einem der vorangegangenen Patentansprüche, **dadurch gekennzeichnet, dass** das Datenverarbeitungssystem (10) mit einem Sender elektromagnetischer Wellen und/oder Sendeempfänger und/oder mit einem Datennetz und/oder einem Rechnernetz verbunden ist.

## Claims

1. Device for the air treatment of at least one room (1) with
- at least one air supply line (2) supplying treated air, each with at least one device for air conditioning (3), each with at least one ionisation module (4) with several ionisation tubes, an air flow sensor (5) and an air humidity sensor (6),
- at least one air exhaust line (7) with at least one air quality sensor (8),
- a data processing system (10) connected to the ionisation module (4), the air flow sensor (5), the air humidity sensor (6), the air quality sensor (8), for increasing or decreasing the ionisation intensity according to the measured values of the air flow sensor (5), the air humidity sensor (6), the air quality sensor (8) and **characterised in that**
- at least one dust measuring device (9) arranged upstream of the ionisation module (4) and/or at least one dust measuring device (11) arranged in the air exhaust line (7) are present, and
- at least one device measuring the load current of the ionisation module (4) and/or at least one ionisation tube of the ionisation module (4), which is or are connected to the data processing system (10), are present
wherein the data processing system (10) is also connected to the dust measuring device (9) upstream of the ionisation module (4) and/or a dust measuring device (11) of the air exhaust line (7), for increasing or decreasing the ionisation intensity according to the measured values of the dust measuring device (9) upstream of the ionisation module (4) and/or the dust measuring device (11) of the air exhaust line (7), the data processing system (10) assigning an operating state of the ionisation module (4) in dependence on the measured values of the dust measuring device (9) in the event of a change in the load current of the ionisation module (4) or at least one ionisation tube, in such a way that a decreasing ionisation power in the event of contamination of at least one ionisation tube can be determined as an operating state of the ionisation module.

2. Device according to claim 1, **characterised in that** the device measuring the load current of an ionisation tube of the ionisation module (4) is connected to the data processing system (10), which, in the event of a change in the load current of the ionisation tube and/or in the event of a change in the load current of the ionisation tube as a function of the measured values of the dust measuring device (9) upstream of the ionisation module (4) and/or the dust measuring device (11) of the air exhaust line (7), is a data processing system (10) associating this change with the operating state of the ionisation tube.

3. Device according to claim 1 or 2, **characterised in that** the air exhaust line (7) of the room (1) is connected to a used air discharge line (12) and/or to an outside air supply line (13), and **in that** the outside air supply line (13) is connected to the device for air conditioning (3).

4. Device according to claim 3, **characterized in that** the used air discharge line (12) has a first fitting (14) and the outside air supply line (13) has a second fitting (15), and **in that** the actuating devices of the fittings (14, 15) for changing the respective air flow are connected to the data processing system (10).

5. Device according to claim 3 or 4, **characterised in that** the outside air supply line (13) has a further dust measuring device (16) for measuring the dust in the outside air and **in that** the dust measuring device (16) in the outside air supply line (13) is connected to the data processing system (10) which, in the event of the occurrence or increased occurrence of dust in the outside air, increases the ionisation power of the ionisation module and/or the air flow as a function of the value of the dust measuring device (16) in the outside air supply line (13).

6. Device according to one of the preceding claims, **characterised in that** the air flow sensor (5), the air humidity sensor (6), the air quality sensor (8) and the dust measuring device (9) upstream of the ionisation module (4) and/or the dust measuring device (11) of the air exhaust line (7) are connected to the data processing system (10) which, as a function of the room size and of measured values of the air flow sensor (5), the air humidity sensor (6), the air quality sensor (8) and the dust measuring device (9) upstream of the ionisation module (4) and/or of the dust measuring device (11) of the air exhaust line (7), determines a room air concentration as a model with weights of the air flow, the air humidity, the air quality and the dust.

7. Device according to one of the preceding claims, **characterized in that** at least one ozone sensor (17) is or are arranged in the air supply line (2) and/or in the air exhaust line (7) and/or in the room (1) and/or in the outside air supply line (13), and **in that** the ozone sensor (17) or the ozone sensors (17) is or are connected with the data processing system (10), which determines the ionisation intensity from the measured values of the air humidity sensor (6), the air flow sensor (5), the air quality sensor (8) and the dust measuring device (9) upstream of the ionisation module (4) and/or the dust measuring device (11) of the air exhaust line (7) and the ozone sensor (17) or the ozone sensors (17) in accordance with the weights of the air humidity, the air flow, the air quality and the dust and an ozone limit value and, in the event of a change in the ionisation intensity, changes the ionisation power in such a way that a compensation and, in accordance with the change, a further reduction or increase in the ionisation intensity takes place, without an impairing or damaging ozone limit value being exceeded.

8. Device according to one of the preceding claims, **characterised in that** at least one carbon dioxide sensor (18) is or are arranged in the air supply line (2) and/or in the air exhaust line (7) and/or in the room (1), and **in that** the carbon dioxide sensor (18) or the carbon dioxide sensors (18) is or are connected to the data processing system (10), which changes the air supply and/or the air flow in the event of a change in the carbon dioxide, taking into account the ionisation intensity, in such a way that the ionisation intensity is determined as a function of the measured values of the air humidity sensor (6), the air flow sensor (5), the air quality sensor (8) and the dust measuring device (9) upstream of the ionisation module (4) and/or the dust measuring device (11) of the air exhaust line (7) and, in the event of a change, a reduction or increase in the ionisation intensity takes place and a carbon dioxide limit value is complied with.

9. Device according to one of the preceding claims, **characterized in that** the ionisation tubes having ionisation module (4) is connected to the data processing system (10) which controls the ionisation power of the ionisation tubes with a pulse width modulation (PWM) by switching periods of the electric grid.

10. Device according to one of the preceding claims, **characterised in that** the device respectively measuring the load current of one ionisation tube of the ionisation module (4) and/or the device measuring the load current of the ionisation tubes of the ionisation module (4) is or are connected to the data processing system (10) in such a way that, in the event of a change in the load current of at least one ionisation tube, the load currents of the other ionisation tubes are changed with respect to a known nominal load current.

11. Device according to one of the preceding claims, **characterised in that** the data processing system (10) is connected to an electromagnetic wave transmitter and/or transceiver and/or to a data network and/or a computer network.

## Revendications

1. Dispositif destiné au traitement de l'air d'au moins une pièce (1), comportant
- au moins une conduite d'amenée d'air (2) amenant de l'air traité et comportant respectivement au moins un dispositif destiné à l'épuration de l'air (3) comportant au moins respectivement un module d'ionisation (4) comportant plusieurs tubes d'ionisation, un capteur de flux d'air (5) et un capteur d'humidité de l'air (6),
- au moins une conduite d'évacuation d'air (7) comportant au moins un capteur de qualité de l'air (8),
- un système de traitement de données (10) connecté au module d'ionisation (4), au capteur de flux d'air (5), au capteur d'humidité de l'air (6) et au capteur de qualité de l'air (8) et permettant d'augmenter ou de réduire l'intensité d'ionisation en fonction des valeurs mesurées du capteur de flux d'air (5), du capteur d'humidité de l'air (6) et du capteur de qualité de l'air (8) et
**caractérisé en ce que**
- au moins un dispositif de mesure de poussière (9) disposé en amont du module d'ionisation (4) et/ou au moins un dispositif de mesure de poussière (11) disposé dans la conduite d'évacuation d'air (7) sont prévus, et
- au moins un dispositif mesurant le courant de charge du module d'ionisation (4) et/ou d'au moins un tube d'ionisation du module d'ionisation (4) est prévu, lequel est connecté au système de traitement de données (10)
dans lequel le système de traitement de données (10) est également connecté au dispositif de mesure de poussière (9) en amont du module d'ionisation (4) et/ou à un dispositif de mesure de poussière (11) de la conduite d'évacuation d'air (7), pour augmenter ou réduire l'intensité d'ionisation en fonction des valeurs mesurées du dispositif de mesure de poussière (9) en amont du module d'ionisation (4) et/ou du dispositif de mesure de poussière (11) de la conduite d'évacuation d'air (7), dans lequel le système de traitement de données (10), en cas de variation de courant de charge du module d'ionisation (4) ou d'au moins un tube d'ionisation, attribue un état de fonctionnement du module d'ionisation (4) en fonction des valeurs mesurées du dispositif de mesure de poussière (9), de telle sorte qu'une puissance d'ionisation décroissante peut être définie comme état de fonctionnement du module d'ionisation en cas de salissure d'au moins un tube d'ionisation.

2. Dispositif selon la revendication de brevet 1, **caractérisé en ce que** le dispositif mesurant le courant de charge d'un tube d'ionisation du module d'ionisation (4) est connecté au système de traitement de données (10), lequel est un système de traitement de données (10) qui, en cas de variation de courant de charge du tube d'ionisation et/ou en cas de variation de courant de charge du tube d'ionisation en fonction des valeurs mesurées du dispositif de mesure de poussière (9) en amont du module d'ionisation (4) et/ou du dispositif de mesure de poussière (11) de la conduite d'évacuation d'air (7), attribue ladite variation à l'état de fonctionnement du tube d'ionisation.

3. Dispositif selon la revendication de brevet 1 ou 2, **caractérisé en ce que** la conduite d'évacuation d'air (7) de la pièce (1) est reliée à une conduite (12) évacuant de l'air vicié et/ou à une conduite (13) amenant l'air ambiant,
**et en ce que** la conduite (13) amenant l'air ambiant est reliée au dispositif destiné à l'épuration de l'air (3).

4. Dispositif selon la revendication de brevet 3, **caractérisé en ce que** la conduite (12) évacuant l'air vicié présente une première armature (14) et la conduite (13) amenant l'air ambiant présente une seconde armature (15),
**et en ce que** les dispositifs d'actionnement des armatures (14, 15) permettant de modifier le flux d'air respectif sont connectés au système de traitement de données (10).

5. Dispositif selon la revendication de brevet 3 ou 4, **caractérisé en ce que** la conduite (13) amenant l'air ambiant possède un dispositif de mesure de poussière (16) supplémentaire permettant de mesurer la poussière dans l'air ambiant, **et en ce que** le dispositif de mesure de poussière (16) dans la conduite (13) amenant l'air ambiant est connecté au système de traitement de données (10), lequel, en présence ou en présence accrue de poussière dans l'air ambiant, augmente, dans la conduite (13) amenant l'air ambiant, la puissance d'ionisation du module d'ionisation et/ou le flux d'air en fonction de la valeur du dispositif de mesure de poussière (16).

6. Dispositif selon l'une des revendications de brevet précédentes, **caractérisé en ce que** le capteur de flux d'air (5), le capteur d'humidité de l'air (6), le capteur de qualité de l'air (8) et le dispositif de mesure de poussière (9) en amont du module d'ionisation (4) et/ou le dispositif de mesure de poussière (11) de la conduite d'évacuation d'air (7) sont connectés au système de traitement de données (10), lequel détermine, en fonction de la taille de la pièce et de valeurs mesurées du capteur de flux d'air (5), du capteur d'humidité de l'air (6), du capteur de qualité de l'air (8) et du dispositif de mesure de poussière (9) en amont du module d'ionisation (4) et/ou du dispositif de mesure de poussière (11) de la conduite d'évacuation d'air (7), une concentration d'air dans la pièce comme modèle comportant les pondérations du flux d'air, de l'humidité de l'air, de la qualité de l'air et de la poussière.

7. Dispositif selon l'une des revendications de brevet précédentes, **caractérisé en ce qu'**au moins un capteur d'ozone (17) est disposé dans la conduite d'amenée d'air (2) et/ou dans la conduite d'évacuation d'air (7) et/ou dans la pièce (1) et/ou dans la conduite (13) amenant l'air ambiant, **et en ce que** le capteur d'ozone (17) ou les capteurs d'ozone (17) sont connectés au système de traitement de données (10), lequel détermine l'intensité d'ionisation à partir des valeurs mesurées du capteur d'humidité de l'air (6), du capteur de flux d'air (5), du capteur de qualité de l'air (8) et du dispositif de mesure de poussière (9) en amont du module d'ionisation (4) et/ou du dispositif de mesure de poussière (11) de la conduite d'évacuation d'air (7) et du capteur d'ozone (17) ou des capteurs d'ozone (17) en fonction des pondérations de l'humidité de l'air, du flux d'air, de la qualité de l'air et de la poussière et d'une valeur limite de concentration d'ozone, et modifie la puissance d'ionisation en cas de variation de l'intensité d'ionisation de telle sorte qu'une compensation et, en fonction de la variation, une réduction ou une augmentation de l'intensité d'ionisation allant au-delà de ladite compensation ont lieu, sans dépassement d'une valeur limite de concentration d'ozone préjudiciable ou nuisible.

8. Dispositif selon l'une des revendications de brevet précédentes, **caractérisé en ce qu'**au moins un capteur de dioxyde de carbone (18) est disposé dans la conduite d'amenée d'air (2) et/ou dans la conduite d'évacuation d'air (7) et/ou dans la pièce (1), **et en ce que** le capteur de dioxyde de carbone (18) ou les capteurs de dioxyde de carbone (18) sont connectés au système de traitement de données (10), lequel modifie, en cas de variation de la concentration de dioxyde de carbone, l'air amené et/ou le flux d'air en tenant compte de l'intensité d'ionisation, de sorte que l'intensité d'ionisation est déterminée en fonction des valeurs mesurées du capteur d'humidité de l'air (6), du capteur de flux d'air (5), du capteur de qualité de l'air (8) et du dispositif de mesure de poussière (9) en amont du module d'ionisation (4) et/ou du dispositif de mesure de poussière (11) de la conduite d'évacuation d'air (7), et, en cas de variation, une réduction ou une augmentation de l'intensité d'ionisation a lieu et une valeur limite de concentration de dioxyde de carbone est respectée.

9. Dispositif selon l'une des revendications de brevet précédentes, **caractérisé en ce que** le module d'ionisation (4) présentant des tubes d'ionisation est connecté au système de traitement de données (10), lequel commande la puissance d'ionisation des tubes d'ionisation par une modulation de largeur d'impulsion (PWM) en commutant des périodes du réseau électrique.

10. Dispositif selon l'une des revendications de brevet précédentes, **caractérisé en ce que** le dispositif mesurant respectivement le courant de charge d'un tube d'ionisation du module d'ionisation (4) et/ou le dispositif mesurant le courant de charge des tubes d'ionisation du module d'ionisation (4) sont connectés au système de traitement de données (10) de sorte que, en cas de variation de courant de charge d'au moins un tube d'ionisation, les courants de charge des autres tubes d'ionisation sont modifiés par rapport à un courant de charge nominal connu.

11. Dispositif selon l'une des revendications de brevet précédentes, **caractérisé en ce que** le système de traitement de données (10) est connecté à un émetteur d'ondes électromagnétiques et/ou émetteur-récepteur et/ou à un réseau de données et/ou un réseau informatique.
